# DEMANDE DE BREVET EUROPEEN

(11) **EP 1 521 082 A2**
(43) Date de publication de la demande: **06.04.2005**
(21) Numéro de dépôt: 04352011.3
(22) Date de dépôt: 30.07.2004
(51) Int. Cl.: G01N 33/543

(54) **Procédé et dispositif de détection et/ou de dosage de molécules**

(30) Priorité: 04.08.2003 FR 0309622
(71) Demandeur: Phylogene SA, 30035 Nimes Cedex (FR)
(72) Inventeur: Skorski, Gilbert, F-30640 Beauvoisin (FR); Aziza, Jean Claude, F-83150 Bandol (FR)
(74) Mandataire: Ravina, Bernard

(57) **Abrégé**

Le dispositif de détection et/ou de dosage (10) de molécules dans un liquide comporte :
- un support (12) comportant au moins un compartiment de réaction (13) dans lequel est placé ledit liquide, et un autre compartiment (14, 15, 16),
- pour chaque molécule recherchée, au moins une microbille magnétique colorée (20) spécifique à ladite molécule recherchée, pour indexer, par la couleur, la présence et/ou la quantité de ladite molécule recherchée,
- un aimant (21) et
- un moyen de déplacement (22) respectif dudit aimant et dudit support pour déplacer chaque microbille magnétique colorée placée dans le champ magnétique dudit aimant depuis le compartiment de réaction dans ledit autre compartiment.

Préférentiellement, le support comporte un disposable (12) comportant au moins le compartiment de réaction (13), au moins un compartiment de révélation (15) et au moins un compartiment de lecture (16), et au moins une zone sèche (17, 18, 19) de séparation d'au moins deux des dits compartiments, chaque compartiment et chaque zone sèche de séparation étant adaptés à recevoir des microbilles magnétiques colorées (20) et à permettre leur déplacement d'un compartiment à un autre, à l'intérieur du disposable.

## Description

La présente invention vise un procédé et un dispositif de détection et/ou de dosage de molécules. Elle s'applique, en particulier, à l'immunodosage et au dosage d'acides nucléiques. Plus particulièrement, la présente invention concerne un procédé et un dispositif de détection multiple (c'est-à-dire de plusieurs molécules, par exemple des acides nucléiques et des protéines) dans le domaine du diagnostic in vitro utilisant des microbilles magnétiques colorées dont la finalité est de générer un signal mesurable.

L'utilisation des microbilles magnétiques dans le domaine des immunodosages ou des acides nucléiques est un procédé connu de longue date. En effet, les microbilles magnétiques permettent d'extraire une molécule cible d'un échantillon et de transporter des microbilles d'un milieu réactionnel à un autre.

L'utilisation de disposables en plastique dans lesquels les réactions sont pratiquées est également pratiqué de longue date car ces objets restent maîtrisables par les fabricants et permettent de garantir le bon déroulement d'un test de diagnostic.

Plus récemment, les systèmes de diagnostic ont évolué vers des solutions qui permettent de détecter plusieurs analytes dans un milieu réactif, ceci étant souvent appelé le multiplexage. Une tendance parallèle est aussi de détecter des analytes en multiplexage dans des volumes de plus en plus réduits, ce qui augmente le potentiel de multiplexage, mais également permet des économies substantielles de réactif.

Aussi, on a vu récemment émerger des dispositifs appelés biopuces, où des réactions sont effectuées sur une surface de verre ou une membrane de polymère, des ligands étant fixés, sous forme de points ou "spot", sur ces supports pour capturer des molécules cibles, lesquelles sont ensuite révélées par d'autres ligands pouvant générer un signal, grâce à un réactif qui réagit simultanément sur tous les spots. La position des différents spots est connue, et la lecture des spots effectuée par exemple par une caméra à dispositif de transfert de charges ou "CCD" qui détecte un changement de couleur d'un spot.

Ces biopuces permettent donc de révéler plusieurs analytes dans un même milieu réactionnel et par là même permettent des économies de réactifs. Le système des biopuces offre de plus l'avantage d'un dispositif jetable ("disposable") permettant une bonne maîtrise de la qualité et une sécurité complémentaire pour l'utilisateur. Mais le système des biopuces souffre de l'inconvénient de manquer de souplesse pour le diagnostic. En effet, la fabrication de chaque biopuce fige la géométrie et la composition des spots. L'analyse requérant rarement tous les analytes fixés, un gaspillage coûteux s'ensuit.

D'autres systèmes, dérivés de la cytométrie de flux, comme celui commercialisé par la société Luminex (marque déposée), utilisent des microbilles colorées sur lesquelles sont fixés des ligands qui capturent des molécules cibles comme décrits ci dessus.

Les microbilles, après réaction, sont envoyées par un flux liquide dans une étroite cellule de lecture et passent devant deux rayons laser. La lecture du signal est effectuée par mesure de la lumière ré-émise par les microbilles qui apportent deux informations sur chaque microbille, la première concernant la couleur, qui est indexée au type de molécule recherchée, et la deuxième, par exemple la fluorescence générée, indiquant la présence de la molécule cible ; de plus, le comptage des microbilles est effectué ce qui permet de savoir combien de microbilles ont détecté quelle molécule recherchée.

Ce système offre une bonne souplesse d'utilisation car le choix de multiplexage peut être effectué avant mise en oeuvre des microbilles, mais il reste toutefois limité à un système de lecture qui est unique et nécessite de nombreux et coûteux lavages entre les comptages des microbilles qui sont indispensables, sinon des contaminations croisées apparaissent.

D'autres évolutions récentes, comme celle de la société Febit (marque déposée) décrite dans le document WO 00/12123 utilisent le même principe précédemment décrit des microbilles colorées, mais associées à un dispositif plastique transparent qui les maintient côte à côte dans un plan, car leur densité ne permet pas une bonne sédimentation. Ce dispositif autorise alors une lecture avec une caméra comme les biopuces. Ce dispositif plastique est associé à une entrée qui conduit vers le dispositif en plan et nécessite une circulation d'un liquide qui va porter les microbilles.

Cette solution combine l'avantage d'une bonne souplesse de définition des analytes du multiplexage associée aux microbilles et l'avantage d'un dispositif jetable contenant les microbilles, le tout étant jetable. Par contre, l'objet plastique nécessite un liquide porteur qui contient les microbilles, et une cellule de lecture qui maintient les microbilles dans un plan ; ceci reste une source de problème associée à la microfluidique et donc aux frottements qui gênent le bon écoulement des microbilles dans la partie en plan.

La présente invention vise à remédier à ces inconvénients et, en particulier à réduire le gaspillage de réactifs, le besoin de lavage et les risques de frottement de billes contre les parois d'une cellule.

A cet effet la présente invention vise, selon un premier aspect, un dispositif de détection et/ou de dosage de molécules dans un liquide, caractérisé en ce qu'il comporte :
- un support comportant au moins un compartiment de réaction dans lequel est placé ledit liquide, et un autre compartiment,
- pour chaque molécule recherchée, au moins une microbille magnétique colorée spécifique à ladite molécule recherchée, pour indexer, par la couleur, la présence et/ou la quantité de ladite molécule recherchée,
- un aimant et
- un moyen de déplacement respectif dudit aimant et dudit support pour déplacer chaque microbille magnétique colorée placée dans le champ magnétique dudit aimant depuis le compartiment de réaction dans ledit autre compartiment.

Grâce à ces dispositions, les molécules recherchées sont détectées en fonction des couleurs des microbilles et leur déplacement est effectuer en déplaçant respectivement l'aimant et le support.

Ainsi, les microbilles magnétiques colorées peuvent successivement servir à effectuer une réaction dans le compartiment de réaction, puis une révélation dans un compartiment de révélation puis une lecture dans un compartiment de lecture sans quitter le support. On observe que certains compartiments peuvent avoir plusieurs fonctions successivement, étant entendu qu'au moins deux compartiments sont prévus dans la dispositif objet de la présente invention.

Les microbilles magnétiques colorées, qui restent dans le champ magnétique de l'aimant sont mises en déplacement par rapport au disposable pour passer d'un compartiment à un autre. On observe que le mouvement respectif de l'aimant par rapport au disposable peut être effectuer en maintenant fixe le disposable et en déplaçant l'aimant et/ou en maintenant fixe l'aimant et en déplaçant le disposable.

Selon des caractéristiques particulières, ledit support comporte un disposable comportant au moins le compartiment de réaction, au moins un compartiment de révélation et au moins un compartiment de lecture, et au moins une zone sèche de séparation d'au moins deux des dits compartiments, chaque compartiment et chaque zone sèche de séparation étant adaptés à recevoir des microbilles magnétiques colorées et à permettre leur déplacement d'un compartiment à un autre à l'intérieur du disposable.

En passant par des zones sèches, les microbilles magnétiques colorées évitent la contamination des bains présents dans les différents compartiments du dispositif.

Selon des caractéristiques particulières, le dispositif tel que succinctement exposé ci-dessus comporte un moyen de commande de déplacement respectif de l'aimant par rapport au disposable.

Grâce à ces dispositions, les opérations peuvent être effectuées automatiquement.

Selon des caractéristiques particulières, le dispositif tel que succinctement exposé ci-dessus comporte un moyen de remplissage et un moyen de purge d'au moins un compartiment.

Grâce à ces dispositions, d'une part, un nettoyage des microbilles magnétiques colorées peut être effectuée en plusieurs bains successivement placés dans le même compartiment et, d'autre part, le même compartiment peut servir à plusieurs étapes du procédé de détection et/ou de dosage.

Selon des caractéristiques particulières, le dispositif tel que succinctement exposé ci-dessus comporte une pluralité de réservoirs contenant, chacun, au moins une microbille magnétique colorée spécifique à une molécule recherchée et un moyen d'amenée sélective de microbilles magnétiques colorées provenant d'une partie de la pluralité de réservoirs.

Grâce à ces dispositions, selon les molécules dont on recherche la détection et/ou le dosage, on met en oeuvre les microbilles magnétiques colorées provenant de différents réservoirs et on évite un gaspillage de réactifs et/ou de microbilles magnétiques colorées.

Selon des caractéristiques particulières, le moyen d'amenée sélective comporte un carrousel sur lequel sont supportés les différents réservoirs. Grâce à ces dispositions, le moyen d'amenée est particulièrement simple à mettre en oeuvre.

Selon des caractéristiques particulières, le dispositif tel que succinctement exposé ci-dessus comporte un bras pipeteur magnétique. Grâce à ces dispositions, les microbilles magnétiques colorées peuvent être prélevées et déplacées de manière aisée et en transportant une faible quantité de liquide.

Selon des caractéristiques particulières, le support est adapté à effectuer une réaction de polymérisation en chaîne connue sous le nom de PCR pour polymerase chain reaction, dans le compartiment de réaction. Grâce à ces dispositions, de faibles quantités de molécules, en particulier d'acides nucléiques, peuvent être mis en oeuvre.

Selon des caractéristiques particulières, le dispositif tel que succinctement exposé ci-dessus comporte un moyen de commande de température d'un compartiment de réaction. Grâce à ces dispositions, une réaction de polymérisation en chaîne peut être effectuée dans un compartiment de réaction.

Selon des caractéristiques particulières, le dispositif tel que succinctement exposé ci-dessus comporte un moyen d'analyse de cinétique d'apparition de signaux lus. Grâce à ces dispositions, la détection et/ou le dosage des molécules recherchées est plus précise.

Selon des caractéristiques particulières, le dispositif tel que succinctement exposé ci-dessus comporte un capteur optoélectronique. Grâce à ces dispositions, la détection et/ou le dosage des molécules recherchées est effectuée par des moyens électroniques, éventuellement numériques, ce qui permet une meilleure fiabilité et une automaticité des résultats.

Selon des caractéristiques particulières, le capteur optoélectronique est une caméra électronique comportant un objectif muni d'un filtre polarisant dont l'axe de polarisation est croisé par rapport à un axe de polarisation de chaque filtre polarisant associé à une source de lumière éclairant le compartiment de lecture.

Selon des caractéristiques particulières, la source de lumière éclairant le compartiment de lecture est un éclairage annulaire centré sur l'axe optique de l'objectif de la caméra.

Grâce à chacune de ces dispositions, les reflets directs sur les microbilles magnétiques colorées sont réduits et l'analyse d'image est améliorée.

Selon un deuxième aspect, la présente invention vise un procédé de détection et/ou de dosage de molécules dans un liquide, caractérisé en ce qu'il comporte :
- une étape de remplissage au cours de laquelle on place ledit liquide dans un compartiment de réaction d'un support comportant au moins ledit compartiment de réaction et un autre compartiment,
- une étape de placement au cours de laquelle, pour chaque molécule recherchée, on place, dans le compartiment de réaction, au moins une microbille magnétique colorée spécifique à ladite molécule recherchée, pour indexer, par la couleur, la présence et/ou la quantité de ladite molécule recherchée et
- une étape de déplacement respectif d'un aimant et dudit support pour déplacer chaque microbille magnétique colorée placée dans le champ magnétique dudit aimant depuis le compartiment de réaction dans ledit autre compartiment.

Selon des caractéristiques particulières, le procédé tel que succinctement exposé ci-dessus comporte une étape de prélèvement sélectif d'au moins une microbille magnétique colorée spécifique à une molécule recherchée dans au moins une partie d'une pluralité de réservoirs contenant, chacun, au moins une microbille magnétique colorée spécifique à une molécule susceptible d'être recherchée dans un liquide.

Selon des caractéristiques particulières, le procédé tel que succinctement exposé ci-dessus comporte une étape de réaction de polymérisation en chaîne connue sous le nom de PCR pour polymerase chain reaction.

Selon des caractéristiques particulières, le procédé tel que succinctement exposé ci-dessus comporte une étape de lecture avec un capteur optoélectronique.

Selon des caractéristiques particulières, le procédé tel que succinctement exposé ci-dessus comporte un étape d'analyse de cinétique d'apparition de signaux lus.

Les avantages, buts et caractéristiques particulières du procédé objet de la présente invention étant similaires à ceux du dispositif objet de la présente invention, ils ne sont pas rappelés ici.

D'autres avantages, buts et caractéristiques de la présente invention ressortiront de la description qui va suivre, faite dans un but explicatif et nullement limitatif en regard des dessins annexés dans lesquels :
- la figure 1 représente, schématiquement, en vue de dessus partielle, un mode de réalisation particulier du dispositif objet de la présente invention,
- la figure 2 représente, schématiquement, en vue en élévation partielle, le mode de réalisation particulier du dispositif objet de la présente invention illustré en figure 1 et
- les figures 3A et 3B représentent un mode de réalisation particulier du procédé objet de la présente invention.

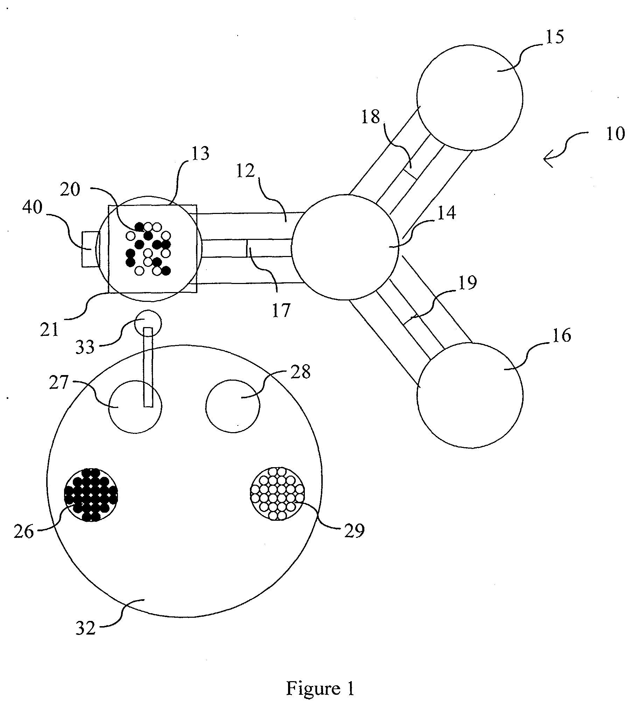

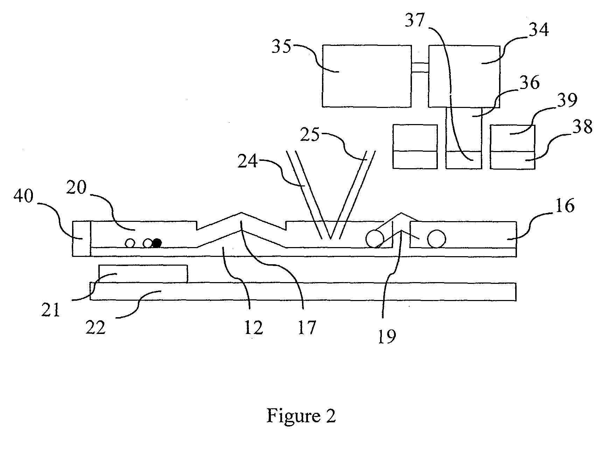

On note que, en figures 1 et 2, les échelles ne sont pas respectées. En particulier, les échelles verticales et horizontales ne sont pas identiques.

On observe, en figures 1 et 2, un dispositif 10 de détection et/ou de dosage de molécules 11, comportant un disposable 12 comportant, lui-même, un compartiment de réaction 13, un compartiment de lavage 14, un compartiment de révélation 15 et un compartiment de lecture 16.

Le disposable 12 est préférentiellement constitué d'un matériau plastique transparent, par exemple du polystyrène ou du métacrylate moulé.

Des zones sèches de séparation 17, 18 et 19 séparent les compartiments de telle manière que les liquides contenus dans ces compartiments ne puissent se mélanger. Les zones sèches 17, 18 et 19 prennent la forme de siphons ou seuils contenant de l'air dans leur partie haute.

Chaque compartiment et chaque zone sèche de séparation est adapté à recevoir des microbilles magnétiques colorées 20 et à permettre leur déplacement d'un compartiment à un autre à l'intérieur du disposable 12.

Des ligands spécifiques aux molécules recherchées, ou molécules cibles sont fixés sur la surface des microbilles magnétiques colorées 20 pour extraire ladite molécule cible, spécifique à la microbille et donc à sa couleur, d'un échantillon, le magnétisme des microbilles permettant de les transporter d'un milieu réactionnel à un autre. Les molécules cibles sont ensuite révélées, dans le compartiment de révélation, par d'autres ligands provoquant une modification de la couleur de la microbille magnétique colorée. La lecture de la détection ou de la quantité de chaque molécule recherchée est ensuite effectuée par analyse de la couleur des microbilles, la couleur étant indexée au type de molécule recherchée et variant en fonction de la présence ou non de la molécule recherchée.

Un aimant mobile 21 est disposé en dessous du disposable 12. Un moyen 22 de déplacement respectif de l'aimant 21 et du disposable 12 assurent le déplacement de l'aimant 21 et/ou du disposable 12 depuis chaque position respective dans laquelle l'aimant 21 est en regard d'un compartiment jusqu'à chaque autre position respective dans laquelle l'aimant 21 est en regard d'un autre compartiment, en passant par une position dans laquelle l'aimant 21 est en regard d'une zone sèche de séparation.

Un moyen de commande (non représenté) de déplacement respectif de l'aimant par rapport au disposable commande les déplacements du moyen de déplacement 22. Le moyen de déplacement 22 est, par exemple, constitué d'un moteur pas-à-pas commandé par un automate ou un ordinateur (non représentés) constituant le moyen de commande.

Un moyen de remplissage 24 et un moyen de purge 25 agissent sur le compartiment de lavage 14 pour, alternativement, le remplir et le vider afin d'effectuer des lavages successifs de microbilles magnétiques colorées décrites ci-dessous.

On observe que les moyens de remplissage et de purge des autres compartiments ne sont pas représentés aux figures, dans un but de clarté de celles-ci. Ils sont, cependant, sensiblement identiques à ceux du compartiment de lavage.

Les moyens de remplissage et de purge sont, par exemple, constitués de pipettes reliées à des pompes pour verser, respectivement aspirer, du liquide dans le compartiment de lavage 14.

Une pluralité de réservoirs 26 à 29 contiennent, chacun, un type de microbilles magnétiques colorées 20, par exemple conservées dans du sérum physiologique. Un moyen d'amenée sélective de microbilles magnétiques colorées provenant d'une partie de la pluralité de réservoirs 26 à 29 comporte un carrousel 32 sur lequel sont supportés les différents réservoirs 26 à 29 et un bras pipeteur magnétique 33. Le carrousel est commandé en rotation par un automate ou un ordinateur (non représenté).

Les microbilles magnétiques colorées 20, constituées selon des techniques connues et possédant des diamètres préférentiellement entre 7 et 50 µm, permettent d'indexer par la couleur le paramètre à doser et d'effectuer un multiplexage souple associé à une lecture des microbilles magnétiques colorées multiplexées, par un capteur optoélectronique 34, par exemple une caméra couleur à dispositif à transfert de charges connu sous le nom de CCD pour charge-coupled device.

Ainsi, l'aimant 21 permet de faire passer les microbilles magnétiques colorées 20 d'un compartiment à un autre du dispositif jetable 12 en franchissant des zones sèches ou seuils séparant les compartiments contenant différents réactifs qui ne doivent pas se mélanger.

De plus, aucun liquide n'est utilisé pour porter les microbilles magnétiques colorées mais les microbilles magnétiques colorées sont mises en déplacement par l'aimant 21 qui les déplace dans les différents compartiments du disposable 12.

De plus, c'est l'aimant 21 qui permet de maintenir les microbilles magnétiques colorées dans un plan contre la paroi du disposable 12, ce qui est préférable pour la lecture par la caméra 34. Cette caractéristique permet également de s'affranchir des problèmes d'écoulement des microbilles magnétiques colorées car il n'y a pas de contrainte géométrique pour les microbilles magnétiques colorées.

Le compartiment de lecture 16 possède, en variante, des positions privilégiées dans lesquelles les microbilles magnétiques colorées se positionnent spontanément. Par exemple, les position privilégiées sont des creux régulièrement espacées formés par moulage dans le fond du compartiment de lecture. Ainsi, les microbilles magnétiques colorées sont espacées les unes des autres et la lecture d'une microbille n'est pas perturbée par les reflets et la couleur des autres microbilles magnétiques colorées.

Le capteur optoélectronique 34 est une caméra électronique comportant un objectif 36 muni d'un filtre polarisant 37 dont l'axe de polarisation est croisé par rapport à un axe de polarisation d'un filtre polarisant 38 associé à une source de lumière 39 éclairant le compartiment de lecture. La source de lumière 39 est, préférentiellement, un éclairage annulaire centré sur l'axe optique de l'objectif 36 de la caméra 34.

Un moyen d'analyse de cinétique d'apparition de signaux lus 35 reçoit les signaux d'image issus de la caméra 34 et les analyse, d'une part, image par image, pour détecter les variations de couleur apparente des microbilles magnétique colorées et, d'autre part, dans la durée, pour traiter la vitesse de ces variations de couleurs.

Par exemple, le moyen d'analyse est constitué d'une carte de numérisation de signaux issus du capteur optoélectronique 34 et un ordinateur (non représenté) mettant en oeuvre un logiciel de traitement d'image approprié.

Le moyen d'analyse 35 détermine ensuite la présence et/ou la quantité de molécules recherchées dans le liquide initialement placé dans le compartiment de réaction.

Dans le mode de réalisation décrit et représenté aux figures, le disposable 12 est adapté à effectuer une réaction de polymérisation en chaîne connue sous le nom de PCR pour polymerase chain reaction. A cet effet, le dispositif 10 comporte un moyen de commande de température 40 du compartiment de réaction 13. Le fonctionnement et les réactifs mis en oeuvre dans la réaction PCR sont connus en soi et ne sont pas décrits ici.

Le moyen de commande de température 40 comporte, par exemple, une résistance dont l'échauffement est commandé par un ordinateur ou un automate (non représentés).

Les différents ordinateurs cités dans la description en regard des figures 1 et 2 sont préférentiellement confondus en un seul ordinateur. De même les différents automates cités dans la description en regard des figures 1 et 2 sont préférentiellement confondus, éventuellement sous la forme d'une carte automate de l'ordinateur précité.

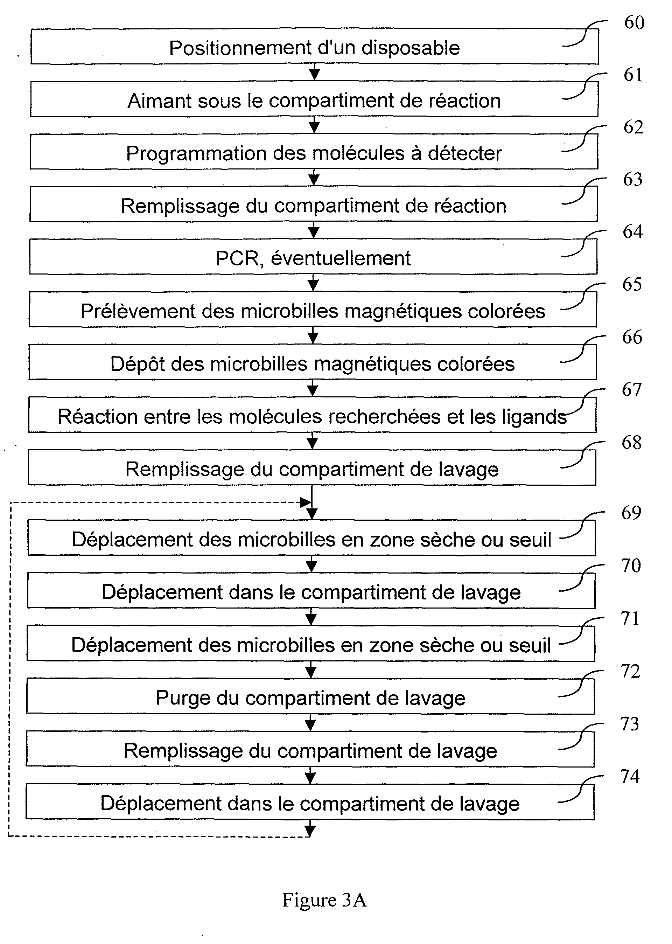

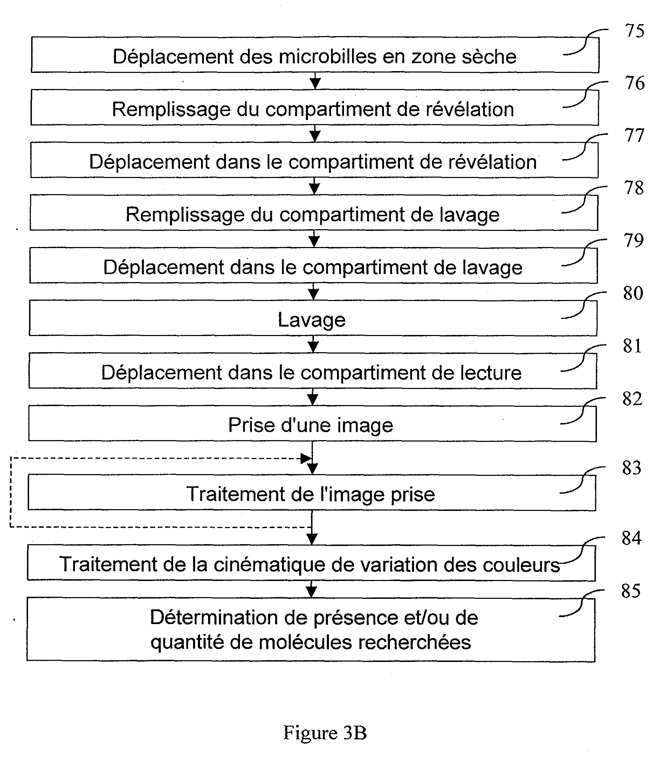

On observe, en figure 3A et 3B, la succession des étapes suivantes :
- positionnement d'un nouveau disposable, étape 60,
- déplacement de l'aimant sous le compartiment de réaction, étape 61,
- programmation des molécules à détecter, étape 62,
- remplissage du compartiment de réaction avec le produit liquide dans lequel les molécules sont recherchées, étape 63,
- PCR, éventuellement, étape 64,
- prélèvement des microbilles magnétiques colorées correspondantes dans les réservoirs, par commande de déplacement du carrousel et du bras pipeteur magnétique, étape 65,
- dépôt des microbilles magnétiques colorées dans le compartiment de réaction par déplacement et inversion de polarité magnétique du bras pipeteur magnétique, étape 66,
- réaction entre le liquide dans lequel des molécules sont recherchées et les ligands des microbilles magnétiques colorées, étape 67,
- remplissage du compartiment de lavage, par exemple avec de l'eau, étape 68,
- déplacement de l'aimant pour déplacer les microbilles magnétiques colorées dans la zone sèche ou seuil séparant le compartiment de réaction du compartiment de lavage, étape 69,
- déplacement de l'aimant pour déplacer les microbilles magnétiques colorées dans le compartiment de lavage, étape 70,
- déplacement de l'aimant pour déplacer les microbilles magnétiques colorées dans la zone sèche ou seuil séparant le compartiment de réaction du compartiment de lavage, étape 71,
- purge du compartiment de lavage, étape 72,
- remplissage du compartiment de lavage, par exemple avec de l'eau, étape 73,
- déplacement de l'aimant pour déplacer les microbilles magnétiques colorées dans le compartiment de lavage, étape 74,
- répétition éventuelle des étapes de lavage (symbolisé par une flèche en traits interrompus),
- déplacement de l'aimant pour déplacer les microbilles magnétiques colorées dans la zone sèche séparant le compartiment de lavage du compartiment de révélation, étape 75 (figure 3B),
- remplissage du compartiment de révélation avec des produits de révélation, étape 76,
- déplacement de l'aimant pour déplacer les microbilles magnétiques colorées dans le compartiment de révélation, étape 77,
- remplissage du compartiment de lavage, par exemple avec de l'eau, étape 78,
- déplacement de l'aimant pour déplacer les microbilles magnétiques colorées dans le compartiment de lavage, étape 79,
- lavage comme indiqué ci-dessus, étape 80,
- déplacement de l'aimant pour déplacer les microbilles magnétiques colorées dans la zone sèche séparant le compartiment de lavage du compartiment de lecture puis dans le compartiment de lecture, étape 81,
- prise d'une image ou lecture des billes dans le compartiment de lecture, étape 82,
- traitement de l'image prise, étape 83,
- répétition des deux dernières étapes, à intervalles de temps, préférentiellement réguliers,
- traitement de la cinématique de variation des couleurs, étape 84 et
- détermination de présence et/ou de quantité de molécules recherchées, étape 85.

On donne, ci-dessous, un exemple de test du dispositif illustré en figures 1 et 2.

Des microbilles colorées magnétiques de diamètre 50 µm et de couleur jaunes et rouges sont revêtues d'une sonde ADN dirigée contre la bactérie E.coli et la bactérie B.subtilis respectivement et spécifiquement.

Une solution 1 contenant de l'ADN amplifié de E.coli est préparée en tampon phosphate pH 5,2, NaCl 0,15 M. Cette solution est obtenue par amplification PCR à l'aide d'un couple de primers, ou amorces, dont un est biotinilé.

Une solution 2 contenant de l'ADN amplifié de B.subtilis est préparée en tampon phosphate pH 5,2, NaCl 0,15 M. Cette solution est obtenue par amplification PCR à l'aide d'un couple de primers, ou amorces, dont un est biotinilé.

Une solution 3 contenant de l'ADN amplifié de B.subtilis et de E. coli est préparée par mélange des deux précédentes.

Un réactif est préparé, contenant un système de révélation constitué d'une enzyme peroxidase, ou marqueur, en solution liée à une molécule de streptavidine, molécule utilisée en PCR.

L'enzyme est révélée par la dégradation d'un substrat tétraméthylbenzidine (TMB) en un dépôt bleu précipitant.

Trois disposables dont la contenance de chaque compartiment est de 200 µl reçoivent chacune environ 50 microbilles jaunes et environ 50 microbilles rouges revêtues de sondes E.coli et B.subtilis respectivement dans 50 µl de solution tampon.

Les trois solutions sont distribuées à raison de 50 µl par compartiment de réaction, chaque disposable ne recevant qu'une solution, et incubées 30 minutes à 60°C.

Un aimant est ensuite appliqué sous le compartiment de réaction et les microbilles sont véhiculées dans le compartiment de lavage, les microbilles étant retenues et déplacées sous l'influence de l'aimant. 100 µl de tampon de lavage sont mis en place et vidés, ou purgés, en utilisant l'aimant pour retenir les microbilles. Cette opération de lavage des microbilles est répétée trois fois.

Les microbilles sont ensuite véhiculées dans le compartiment de révélation où l'enzyme liée à la streptavidine est mise en place à raison de 50 µl par compartiment de révélation et incubée 30 minutes à 37°C.

Après lavage comme précédemment, dans le compartiment de lavage, 50 µl de TMB est distribué par compartiment de révélation.

Les microbilles magnétiques sont ensuite véhiculées dans le compartiment de lecture, où elles se positionnent toutes dans le même plan au fond du compartiment de lecture sous l'action de l'aimant et le compartiment de lecture est ensuite lu par le dessus à intervalle de temps régulier pendant 30 minutes par une caméra électronique. Les images sont analysées et traitées, tant une à une que dans la progression des variations de couleurs et permettent de mettre en évidence les résultats, c'est à dire la détection ou le dosage des molécules recherchées, par la modification des couleurs.

Les ADNs biotinilés s'hybrident avec les sondes qui leur correspondent sur les microbilles de couleur correspondante. Les ADNs hybridés vont ensuite par affinité entre la biotine et la streptavidine, retenir l'enzyme qui permet un dépôt local de la couleur bleue, induisant une modification de couleur de la microbille.

Le premier disposable contient des microbilles jaunes et des microbilles rouges sombre à violettes, ce qui correspond à la révélation de la solution d'ADN de B. subtilis. La précipitation de substrat bleu de TMB modifie la couleur des microbilles rouges en rouge sombre/violet révélant la présence de B. subtilis.

Le deuxième disposable contient des microbilles vertes et des microbilles rouges, ce qui correspond à la révélation d'ADN de E. coli. La précipitation de substrat bleu de TMB modifie la couleur des microbilles jaunes en vert révélant la présence de B. subtilis.

Le troisième disposable contient des microbilles vertes et des microbilles rouges, ce qui correspond à la révélation d'ADN de E. coli et de B. subtilis. La précipitation de substrat bleu de TMB modifie la couleur des microbilles jaunes en vert et de microbille rouges en rouge sombre/violet révélant la présence de B. subtilis et de E.coli.

La présente invention s'applique, en particulier, à l'immunodosage et au dosage d'acides nucléiques, en particulier pour des détections multiples (c'est-à-dire de plusieurs molécules, par exemple des acides nucléiques et des protéines). Elle trouve des applications dans le domaine du diagnostic in vitro.

## Revendications

1. Dispositif de détection et/ou de dosage (10) de molécules dans un liquide, **caractérisé en ce qu'**il comporte :
- un support (12) comportant au moins un compartiment de réaction (13) dans lequel est placé ledit liquide, et un autre compartiment (14, 15, 16),
- pour chaque molécule recherchée, au moins une microbille magnétique colorée (20) spécifique à ladite molécule recherchée, pour indexer, par la couleur, la présence et/ou la quantité de ladite molécule recherchée,
- un aimant (21) et
un moyen de déplacement (22) respectif dudit aimant et dudit support pour déplacer chaque microbille magnétique colorée placée dans le champ magnétique dudit aimant depuis le compartiment de réaction dans ledit autre compartiment.

2. Dispositif selon la revendication 1, **caractérisé en ce que** ledit support comporte un disposable (12) comportant au moins le compartiment de réaction (13), au moins un compartiment de révélation (15) et au moins un compartiment de lecture (16), et au moins une zone sèche (17, 18, 19) de séparation d'au moins deux des dits compartiments, chaque compartiment et chaque zone sèche de séparation étant adaptés à recevoir des microbilles magnétiques colorées (20) et à permettre leur déplacement d'un compartiment à un autre, à l'intérieur du disposable.

3. Dispositif selon l'une quelconque des revendications 1 ou 2, **caractérisé en ce qu'**il comporte un moyen de commande de déplacement respectif de l'aimant par rapport au disposable.

4. Dispositif selon l'une quelconque des revendications 1 à 3, **caractérisé en ce qu'**il comporte un moyen de remplissage (24) et un moyen de purge (25) d'au moins un compartiment.

5. Dispositif selon l'une quelconque des revendications 1 à 4, **caractérisé en ce qu'**il comporte une pluralité de réservoirs (26 à 29) contenant, chacun, au moins une microbille magnétique colorée spécifique à une molécule recherchée et un moyen d'amenée sélective (32, 33) de microbilles magnétiques colorées provenant d'une partie de la pluralité de réservoirs.

6. Dispositif selon la revendication 5, **caractérisé en ce que** le moyen d'amenée sélective comporte un carrousel (32) sur lequel sont supportés les différents réservoirs (26 à 29).

7. Dispositif selon l'une quelconque des revendications 1 à 6, **caractérisé en ce qu'**il comporte un bras pipeteur magnétique (33).

8. Dispositif selon l'une quelconque des revendications 1 à 7, **caractérisé en ce que** le support (12) est adapté à effectuer une réaction de polymérisation en chaîne connue sous le nom de PCR pour polymerase chain reaction, dans le compartiment de réaction (13).

9. Dispositif selon l'une quelconque des revendications 1 à 8, **caractérisé en ce qu'**il comporte un moyen de commande de température (40) d'un compartiment de réaction (13).

10. Dispositif selon l'une quelconque des revendications 1 à 9, **caractérisé en ce qu'**il comporte un moyen d'analyse de cinétique d'apparition de signaux lus (35).

11. Dispositif selon l'une quelconque des revendications 1 à 10, **caractérisé en ce qu'**il comporte un capteur optoélectronique (34).

12. Dispositif selon la revendication 11, **caractérisé en ce que** le capteur optoélectronique (34) est une caméra électronique comportant un objectif (36) muni d'un filtre polarisant (37) dont l'axe de polarisation est croisé par rapport à un axe de polarisation de chaque filtre polarisant (38) associé à une source de lumière (39) éclairant le compartiment de lecture.

13. Dispositif selon la revendication 12, **caractérisé en ce que** la source de lumière (39) éclairant le compartiment de lecture est un éclairage annulaire centré sur l'axe optique de l'objectif (36) de la caméra (34).

14. Procédé de détection et/ou de dosage de molécules dans un liquide, **caractérisé en ce qu'**il comporte :
- une étape de remplissage (63) au cours de laquelle on place ledit liquide dans un compartiment de réaction (13) d'un support (12) comportant au moins ledit compartiment de réaction et un autre compartiment (14, 15, 16),
- une étape de placement (65, 66) au cours de laquelle, pour chaque molécule recherchée, on place, dans le compartiment de réaction, au moins une microbille magnétique colorée (20) spécifique à ladite molécule recherchée, pour indexer, par la couleur, la présence et/ou la quantité de ladite molécule recherchée et
- une étape de déplacement (69, 70, 71, 74, 75, 77, 79, 81) respectif d'un aimant (21) et dudit support pour déplacer chaque microbille magnétique colorée placée dans le champ magnétique dudit aimant depuis le compartiment de réaction dans ledit autre compartiment.

15. Procédé selon la revendication 14, **caractérisé en ce qu'**il comporte une étape de prélèvement sélectif (65) d'au moins une microbille magnétique colorée, spécifique à une molécule recherchée dans au moins une partie d'une pluralité de réservoirs (26 à 29) contenant, chacun, au moins une microbille magnétique colorée spécifique à une molécule susceptible d'être recherchée dans un liquide.

16. Procédé selon l'une quelconque des revendications 14 ou 15, **caractérisé en ce qu'**il comporte une étape de réaction de polymérisation en chaîne (64) connue sous le nom de PCR pour polymerase chain reaction.

17. Procédé selon l'une quelconque des revendications 14 à 16, **caractérisé en ce qu'**il comporte une étape de lecture (82, 83) avec un capteur optoélectronique (34).

18. Procédé selon l'une quelconque des revendications 14 à 17, **caractérisé en ce qu'**il comporte un étape d'analyse de cinétique d'apparition de signaux lus (84).
